# EUROPEAN PATENT APPLICATION

(11) **EP 4 393 498 A1**
(43) Date of publication of application: **03.07.2024**
(21) Application number: 23194762.3
(22) Date of filing: 01.09.2023
(51) Int. Cl.: A61K 35/19, A61K 35/16, A01N 1/02, C12N 5/078, A61P 17/00, A61P 19/00

(54) **PLATELETS-CONTAINING COMPOSITION AND PREPARATION PROCESS THEREOF**

(30) Priority: 30.12.2022 TW 111150999
(71) Applicant: Spirit Scientific Co. LTD., New Taipei City 221416 (TW); Lin, Dao Lung, Steven, New Taipei City 22175 (TW)
(72) Inventor: Chen, Chin-Ho, 221416 New Taipei City (TW); Lin, Dao-Lung, 221416 New Taipei City (TW)
(74) Representative: Scholz, Volker

(57) **Abstract**

The present disclosure provides a platelets-containing composition and the preparation process thereof. The platelets-containing composition includes a quantified and activated platelet-rich plasma and a platelet-poor plasma mixed with the quantified and activated platelet-rich plasma. The preparation process of the platelets-containing composition is to purify and quantify platelets from the collected whole blood to produce the platelets-containing composition with high purity, which can have the best growth factor sustained-release effect.

## Description

### CROSS-REFERENCE TO RELATED APPLICATION

This application claims priority of Taiwan patent application No. 111150999, filed on December 30, 2022, the content of which is incorporated herein in its entirety by reference.

### BACKGROUND OF THE INVENTION

### 1. Field of the Invention

The present invention relates to a composition and a process, which refer to a product obtained by mixing platelet-rich plasma (PRP) and platelet-poor plasma (PPP), followed by freeze-drying and performing sterilization, and a platelets-containing composition prepared there from and the preparation process of the platelets-containing composition.

### 2. The Prior Art

The traditional platelet product involves centrifuging whole blood, and then using plasma extractor or automatic platelet machine to collect platelet-rich plasma (PRP). However, without a quantitative procedure, the platelet counts in platelet-rich plasma (PRP) cannot be estimated accurately. The traditional preparation of platelet composition is to add other ingredients, such as collagen, calcium ions, etc., to the platelet-rich plasma (PRP), and creating liquid, powder, colloid forms, etc. It cannot rule out allergens, toxicity and other doubts. The traditional process of forming platelet gel requires additional heating (generally at 37°C), which is inconvenient to use and cannot avoid the problem of heat damage to platelet activity. The traditional platelet gel needs to mix with other substances such as gelatin, chitosan, hyaluronic acid, etc., to form a colloid. The added substances may destroy or impair platelet activity.

The applicant searches through the following keywords, such as: (platelet lysate) (gel), (~patent/US20120183519A1), (~patent/US20190160104A1), (~patent/US20120183519A1) (sterile), (~patent/CN110876815A) (platelet lysate), (~patent/CN110876815A) (platelet) (gel); 8 prior arts were screened out as follows.

As disclosed in prior art 1: U.S. patent "Treatment of erectile dysfunction using platelet-rich plasma" (Publication No.: US20120183519A1), a method of treating erectile dysfunction in a subject, involves administering a composition comprising platelet-rich plasma at or proximate to ischemic tissue comprising the corpora cavernosa or corpus spongiosum. However, this application has limitations as uses platelet-rich plasma (PRP) direct injection for treatment, which poses a risk of loss after the injection is completed, and prevents the growth factor from being slowly released for treatment. Moreover, it cannot be sterilized and has the possibility of potential infection.

As disclosed in prior art 2: U.S. patent "Platelet lysate gel" (Publication No.: US20190160104A1), a human platelet lysate is produced according to a method comprising subjecting a population of human platelets to four freeze-thaw cycles, wherein the freeze portion of each cycle is carried out at a temperature lower than or equal to -190° C. However, this method involves producing platelet-rich plasma (PRP) first, and then adding a polymer to complete the preparation, and the polymer may be toxic.

As disclosed in prior art 3: CN patent "Hydrogel loaded with platelet-rich plasma and antibacterial peptide as well as preparation method and application of hydrogel" (Publication No.: CN110876815A), method of producing a hydrogel involves producing platelet-rich plasma (PRP) first, and then mixing the platelet-rich plasma (PRP) with antimicrobial peptides and synthetic polymers. The polymer is prepared by chemical synthesis, dialysis and freeze-drying. However, according to the content of the Material Safety Data Sheet, it is reported to be irritating and unsuitable for use on wounds.

As disclosed in prior art 4: CN patent "Compositions, uses, and preparation of platelet lysates" (Patent No.: CN103702674B). After the liquid is obtained by extrusion and centrifugation, the colloid is discarded, and only the processed liquid is retained, which cannot be effectively applied to the treatment of more types of wounds.

As disclosed in prior art 5: CN patent "Viral inactivated platelet extract, use and preparation thereof' (Publication No.: CN108714155A), a method of preparing platelet extract involves using an organic solvent for sterilization, and removing the solvent which makes the steps complicated and creates a residual risk.

As disclosed in prior art 6: CN patent "PROCESS FOR PREPARING A POOLED HUMAN PLATELET LYSATE, POOLED HUMAN PLATELET LYSATE AND ITS USE FOR TREATING NEUROLOGICAL DISORDERS" (Publication No.: CN110891582A), a method of preparing platelet lysate involves processing it at a higher temperature and for a longer time before using it, and having a complicated, use procedure, and requiring cooling before using it.

As disclosed in prior art 7: CN patent "Closed quick blood platelet lysis solution preparation device" (Patent No.: CN204446735U), a device for rapid preparation of platelet lysate is provided. However, the platelet lysate prepared by this device cannot be preserved, and requires preparation again every time it is needed to be used.

As disclosed in prior art 8: CN patent "Compound platelet gel and preparation method thereof " (Patent No.: CN105030826A), a method of preparing platelet gel involves adding chitosan and glucose, and having more additives that would easily lead to more safety problems.

### SUMMARY OF THE INVENTION

To solve the above-mentioned deficiencies in the prior art, the present invention provides a platelets-containing composition, comprising: a quantified and activated platelet-rich plasma (PRP) mixed with platelet-poor plasma (PPP), which overcomes the difficulties in the prior art.

The secondary object of the present invention is to provide a method for preparing a platelets-containing composition, comprising the following steps: collecting whole blood; performing centrifugation to separate platelet-rich plasma (PRP) and platelet-poor plasma (PPP); quantifying and activating the platelet-rich plasma (PRP) into highly concentrated platelet plasma (HCPP); mixing the HCPP with the platelet-poor plasma (PPP); freeze-drying; sterilization; and completing the preparation of the platelets-containing composition.

Another object of the present invention is to provide a method for preparing a platelets-containing composition. The platelets-containing composition has the following advantages: it can be stored at room temperature, without adding additional complexes; it can be used without heating, and it can be prepared in any shape and thickness of high-purity platelets-containing composition.

Another object of the present invention is to provide a platelets-containing composition and the preparation process thereof, which can effectively improve its ease of use, safety, and have the best growth factor sustained-release results.

The problem to be solved by the present invention is as follows: in contrast to the traditional platelet gel, it can only be applied externally on the patient's wound, thus achieving the effect of "protection". Preparation using non-patient's own blood collection, it cannot avoid issues of rejection, allergens, toxicity, and cannot make the wound repair faster, and there is a risk of infection. The traditional preparation of platelet composition involves adding other ingredients, such as collagen, calcium ions, etc., to platelet-rich plasma (PRP), and creating liquid, powder, colloid forms, etc. It cannot eliminate concerns about allergens, toxicity. The traditional platelet gel gelation process requires additional heating (generally at 37°C), which is inconvenient to use and cannot lead the problem of heat damage to platelet activity. The traditional platelet gel needs mix with other substances such as gelatin, chitosan, hyaluronic acid, etc., to form a colloid. The added substances may adversely affect platelet activity.

According to prior art 1: it is a U.S. patent: "Treatment of erectile dysfunction using platelet-rich plasma" (Publication No.: US20120183519A1). The adaptability of the prior art 1 is to use platelet-rich plasma (PRP) direct injection for treatment, there is a risk of losing the injected is completed, and the growth factor cannot be slowly released for treatment. At the same time, it cannot be sterilized and has a potential risk of infection. According to prior art 2: it is a U.S. patent: "Platelet lysate gel" (Publication No.: US20190160104A1). The prior art 2 is to produce platelet-rich plasma (PRP) first, and then add a polymer to complete the preparation, and the polymer may be toxic. According to prior art 3: it is a CN patent: "Hydrogel loaded with platelet-rich plasma and antibacterial peptide as well as preparation method and application of hydrogel" (Publication No.: CN110876815A), prior art 3 is to produces platelet-rich plasma (PRP) first, and then mixes the platelet-rich plasma (PRP) with antimicrobial peptides and synthetic polymers, the polymer is prepared by chemical synthesis, dialysis and freeze-drying. According to the content of the Material Safety Data Sheet, it is reported to be irritating and unsuitable for use on wounds. According to prior art 4: it is a CN patent: "Compositions, uses, and preparation of platelet lysates" (Patent No.: CN103702674B). The colloid in the prior art 4 is discarded, it cannot be effectively applied to treat more types of wounds. According to prior art 5: it is a CN patent: "Viral inactivated platelet extract, use and preparation thereof" (Publication No.: CN108714155A), it uses an organic solvent for sterilization, and the solvent needs to be removed, the steps are complicated and there is a residual risk. According to prior art 6: it is a CN patent: "PROCESS FOR PREPARING A POOLED HUMAN PLATELET LYSATE, POOLED HUMAN PLATELET LYSATE AND ITS USE FOR TREATING NEUROLOGICAL DISORDERS" (Publication No.: CN110891582A). The prior art 6 requires a higher temperature to be used, and the use procedure is complicated, and it needs to be cooled before it can be used. According to prior art 7: it is a CN patent: "Closed quick blood platelet lysis solution preparation device" (Patent No.: CN204446735U). The lysis solution prepared by this device cannot be preserved, and it needs to be prepared again every time it needs to be used. According to prior art 8: it is a CN patent: "Compound platelet gel and preparation method thereof " (Patent No.: CN105030826A). It needs to add additional substances such as chitosan and glucose, which may lead to more safety concerns.

To achieve the above objects, the present invention provides a platelets-containing composition, characterized in that the platelets-containing composition comprises: at least one platelet-rich plasma (PRP) that has been quantified and activated; at least one platelet-poor plasma (PPP), wherein the at least one PPP is mixed with the quantified and activated platelet-rich plasma (PRP) to form a mixture, and the mixture is freeze-dried and sterilized to form the platelets-containing composition with high purity and sustained release of growth factors.

According to an embodiment of the present invention, a platelets-containing composition can be added with an additive according to the use requirement, so that the platelets-containing composition can be used in colloid or liquid form.

To achieve the above objects, the present invention provides a method for preparing a platelets-containing composition, comprising the following steps: 1. collecting whole blood; 2. performing centrifugation to separate platelet-rich plasma (PRP) and platelet-poor plasma (PPP); 3. quantifying and activating the platelet-rich plasma (PRP) into highly concentrated platelet plasma (HCPP); 4. mixing the HCPP with the platelet-poor plasma (PPP); 5. freeze-drying; 6. performing sterilizing; 7. completing the preparation of the platelets-containing composition.

Wherein, in step 7, a platelets-containing composition can be added with an additive according to the use requirement, so that the platelets-containing composition can be used in colloid or liquid form.

According to an embodiment of the present invention, the additive is in the range of 0.01-10% Ca²⁺, and to form a platelet gel

Preferably, additive added per milliliter (mL) of the platelets-containing composition is in the range of 0.05-9.75% Ca²⁺.

Preferably, additive added per milliliter (mL) of the platelets-containing composition is in the range of 0.1-9% Ca²⁺.

Preferably, additive added per milliliter (mL) of the platelets-containing composition can be the group consisting of: 0.01%, 0.02%, 0.03%, 0.04%, 0.05%, 0.06%, 0.07%, 0.08%, 0.09%, 0.1%, 0.11%, 0.12%, 0.13%, 0.14%, 0.15%, 0.16%, 0.17%, 0.18%, 0.19%, 0.2%, 0.21%, 0.22%, 0.23%, 0.24%, 0.25%, 0.26%, 0.27%, 0.28%, 0.29%, 0.3%, 0.31%, 0.32% , 0.33%, 0.34%, 0.35%, 0.36%, 0.37%, 0.38%, 0.39, 0.4%, 0.41%, 0.42%, 0.43%, 0.44%, 0.45%, 0.46%, 0.47%, 0.49%, 0.5% , 0.51%, 0.52%, 0.53%, 0.54%, 0.55%, 0.56%, 0.57%, 0.58%, 0.59%, 0.6%, 0.61%, 0.62%, 0.63%, 0.64%, 0.65%, 0.66%, 0.67 %, 0.68%, 0.69%, 0.7%, 0.71%, 0.72%, 0.73%, 0.74%, 0.75%, 0.76%, 0.77%, 0.78%, 0.79%, 0.8%, 0.81%, 0.82%, 0.83%, 0.84%, 0.85%, 0.86%, 0.87%, 0.89%, 0.9%, 1%, 1.25%, 1.5%, 1.75%, 2%, 2.25%, 2.5%, 2.75%, 3%, 3.25%, 3.5% , 3.75%, 4%, 4.25%, 4.5%, 4.75%, 5%, 5.25%, 5.75%, 6%, 6.25%, 6.5%, 6.75%, 7%, 7.25%, 7.5%, 8.75%, 9 %, 9.25%, 9.5%, 9.75%, and 10%.

According to an embodiment in step 7 of the present invention, the gel can be prepared in shape and thickness according to actual requirement, making it suitable for irregular wounds, or as a repair and protection on a bone.

According to an embodiment of the present invention, an additive is a solvent such as water for injection, and a platelet composite solution is formed.

According to an embodiment of the present invention, the sterilization in step 6 can be performed by methods such as gamma ray and E-Beam.

According to an embodiment of the present invention, quantifying the platelet-rich plasma (PRP) in step 3 is by centrifuging and adding water for injection to obtain platelet concentration to 10⁹/mL.

According to an embodiment of the present invention, the quantification of the platelet-rich plasma (PRP) can also be achieved by adding sterile normal saline, 0.45% NaCl, 4.5% hypertonic saline, sterile hot spring water or isotonic saline as a substitute for water for injection.

According to an embodiment of the present invention, activating platelet-rich plasma (PRP) in step 3 is by adding an activator or by ultrasonic vibration.

According to an embodiment of the present invention, activating platelet-rich plasma (PRP)in step 3 is by adding an activator, and supernatant is removed by centrifugation to mix with platelet-poor plasma (PPP).

According to an embodiment of the present invention, activating platelet-rich plasma (PRP) in step 3 includes, but is not limited to ultrasonic vibration to mix with platelet-poor plasma (PPP).

Compared with the prior art, the platelets-containing composition of the present invention consists of quantified and activated platelet-rich plasma (PRP) mixed with platelet-poor plasma (PPP). The method for preparing the platelets-containing composition includes the following steps: collecting whole blood; centrifuging to separate platelet-rich plasma (PRP) and platelet-poor plasma (PPP); quantifying and activating the platelet-rich plasma (PRP) into highly concentrated platelet plasma (HCPP); mixing the HCPP and the platelet-poor plasma (PPP); freeze-drying; sterilizing; and obtaining the platelets-containing composition. The platelets-containing composition can be stored at room temperature, does not need additional complexes or heating before use, and can be into platelets-containing any shape and thickness of high-purity platelet-containing composition. The platelets-containing composition and its preparation process can effectively improve its usability, safety, and growth factor sustained-release results. It will greatly expand the industrial applications and have novelty and inventive step.

### BRIEF DESCRIPTION OF THE DRAWINGS

FIG. 1: Structural diagram of the platelets-containing composition of the present invention.
FIG. 2: The preparation flow chart of the method for preparing the platelets-containing composition of the present invention.
FIG. 3: The flow chart of the use of platelet gel and platelet composite solution according to the present invention.

### DETAILED DESCRIPTION OF THE PREFERRED EMBODIMENT

The accompanying drawings illustrate the specific embodiments in which the present disclosure may be practiced. These embodiments are provided to enable those skilled in the art to practice the present disclosure. Other embodiments may be used and changes can be made to the embodiments without departing from the scope of the present invention. The following description is therefore not intended to limit the scope of the present invention.

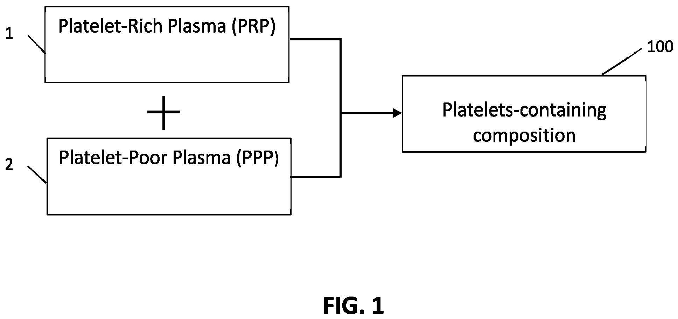

Referring to FIG. 1, is a structural diagram of a platelets-containing composition of the present invention. In a preferred embodiment, the platelets-containing composition 100 of the present invention comprises at least one platelet-rich plasma (PRP) 1 and at least one platelet-poor plasma (PPP) 2.

The aforementioned at least one platelet-rich plasma (PRP) 1 is obtained by collecting whole blood through centrifugation and extracting the middle layer. The platelet-rich plasma (PRP) 1 is then quantified and activated.

The aforementioned at least one platelet-poor plasma (PPP) 2 is obtained from whole blood by centrifugation and taking the upper layer.

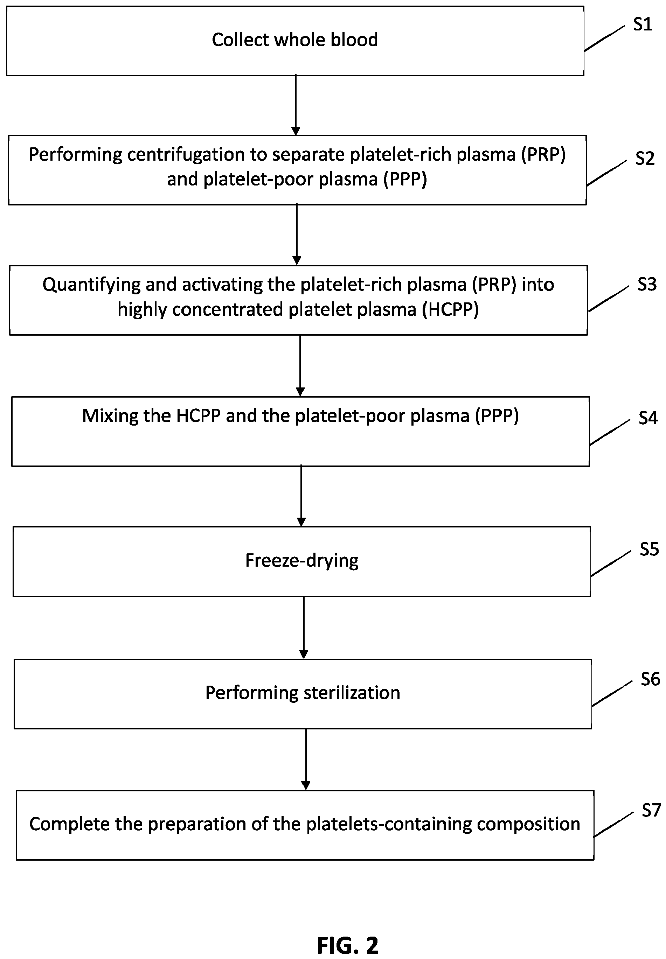

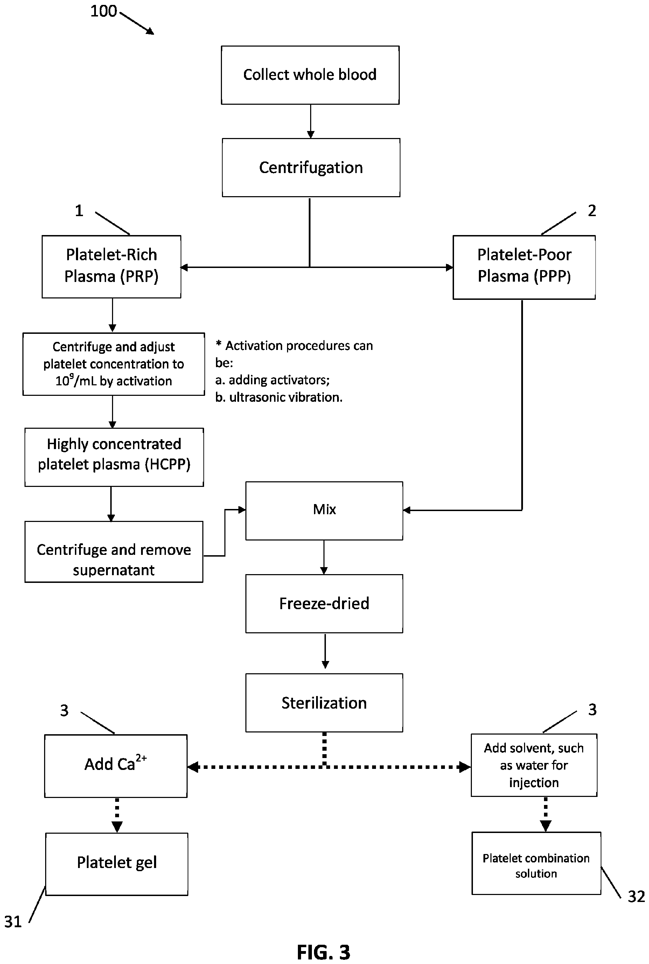

Referring to FIG. 2 and FIG. 3, which are the preparation flow chart of the method for preparing the platelets-containing composition of the present invention, and the flow chart of the implementation of platelet gel and platelet composite solution of the present invention. In a preferred embodiment of the method for preparing the platelets-containing composition of the present invention, the core technology of the present invention (as shown in FIG. 2) is to provide a method for preparing a platelets-containing composition, comprising the following steps.
1. Collect whole blood by S1. (e.g., taking blood from a blood bag) (as shown in FIG. 2).
2. Performing centrifugation to separate platelet-rich plasma (PRP) and platelet-poor plasma (PPP) by S2 (as shown in FIG. 2).
3. Quantifying and activating the platelet-rich plasma (PRP) into highly concentrated platelet plasma (HCPP) by S3 (as shown in FIG. 2).
4. Mixing the HCPP and the platelet-poor plasma (PPP) by S4 (as shown in FIG. 2).
5. Freeze-drying by S5 (as shown in FIG. 2).
6. Performing sterilization by S6 (as shown in FIG. 2).
7. Complete the preparation of the platelets-containing composition 100 by S7.

In step 7, the platelets-containing composition 100 can be added with an additive 3 according to requirement of use, so that the platelets-containing composition 100 can be used in colloid or liquid form. The additive 3 is in the range of 0.01-10% Ca²⁺, and to form a platelet gel 31. Preferably, additive 3 added per milliliter (mL) of the platelets-containing composition 100 is in the range of 0.05-9.75% Ca²⁺. Preferably, additive added per milliliter (mL) of the platelets-containing composition 100 is in the range of 0.1-9% Ca²⁺. Preferably, additive added per milliliter (mL) of the platelets-containing composition 100 can be the group consisting of: 0.01%, 0.02%, 0.03%, 0.04%, 0.05%, 0.06%, 0.07%, 0.08%, 0.09%, 0.1%, 0.11%, 0.12%, 0.13%, 0.14%, 0.15%, 0.16%, 0.17%, 0.18%, 0.19%, 0.2%, 0.21%, 0.22%, 0.23%, 0.24%, 0.25%, 0.26%, 0.27%, 0.28%, 0.29%, 0.3%, 0.31%, 0.32% , 0.33%, 0.34%, 0.35%, 0.36%, 0.37%, 0.38%, 0.39, 0.4%, 0.41%, 0.42%, 0.43%, 0.44%, 0.45%, 0.46%, 0.47%, 0.49%, 0.5% , 0.51%, 0.52%, 0.53%, 0.54%, 0.55%, 0.56%, 0.57%, 0.58%, 0.59%, 0.6%, 0.61%, 0.62%, 0.63%, 0.64%, 0.65%, 0.66%, 0.67 %, 0.68%, 0.69%, 0.7%, 0.71%, 0.72%, 0.73%, 0.74%, 0.75%, 0.76%, 0.77%, 0.78%, 0.79%, 0.8%, 0.81%, 0.82%, 0.83%, 0.84%, 0.85%, 0.86%, 0.87%, 0.89%, 0.9%, 1%, 1.25%, 1.5%, 1.75%, 2%, 2.25%, 2.5%, 2.75%, 3%, 3.25%, 3.5% , 3.75%, 4%, 4.25%, 4.5%, 4.75%, 5%, 5.25%, 5.75%, 6%, 6.25%, 6.5%, 6.75%, 7%, 7.25%, 7.5%, 8.75%, 9 %, 9.25%, 9.5%, 9.75%, and 10%. The gel can be prepared in shape and thickness of actual requirement to, making it suitable for irregular wounds, or as a repair and protection on a bone. The additive 3 is water for injection, and a platelet composite solution is formed. The sterilization in step 6 includes, but is not limited to gamma ray and E-Beam. Quantifying and activating the platelet-rich plasma (PRP) in step 3 is through centrifugation and adding water for injection to adjust platelet concentration to 10⁹/mL. The quantification of the platelet-rich plasma (PRP) can also be achieved by adding sterile normal saline, 0.45% NaCl, 4.5% hypertonic saline, sterile hot spring water or isotonic saline. Activating the platelet-rich plasma (PRP) 1 in step 3 is by adding an activator or by ultrasonic vibration (as shown in FIG. 3). Activating the platelet-rich plasma (PRP) 1 in step 3 includes but is not limited to adenosine diphosphate (ADP), for creating and maintaining platelets in an activated state, followed by removal the activator, and then achieving the effect of higher platelet purity and growth factor content. Activating highly concentrated platelet plasma (HCPP) in step 3 is through, but not limited to ultrasonic vibration to mix with platelet-poor plasma (PPP) 2 (as shown in FIG. 3). Activating the PRP 1 in step 3 is by adding an activator, and supernatant is removed by centrifugation to mix the HCPP and the PPP 2. Activating the PRP 1 in step 3is through ultrasonic vibration to mix the HCPP and the PPP 2 (as shown in FIG. 3).

Referring to FIG. 3, which is the flow chart of the preparation process of platelet gel and platelet composite solution of the present invention. The preparation process of the platelets-containing composition takes "platelet gel" as an example, comprising the following steps. Whole blood was collected first, and PRP and PPP were prepared by centrifugation. PRP was then centrifuged and the platelet concentration was quantitatively adjusted to 10⁹/mL. Water for injection can be added to the quantitative procedure, and the previously quantified PRP can be activated by ultrasonic vibration or added with the activator to achieve platelet purity of 100%, making the growth factor content have a higher effect. The aforementioned activation procedure and quantitative HCPP were centrifuged and the supernatant was removed, and the activated HCPP were mixed with the aforementioned PPP obtained by centrifugation, lyophilized, and then sterilized by gamma ray. Finally, 0.01-10% Ca2⁺ of additive 3 was added to form a platelet gel 31.

Referring to FIG. 3, which is the flow chart of the preparation process of platelet gel and platelet composite solution of the present invention. The preparation process of the platelets-containing composition takes "platelet composite solution" as an example, comprising the following steps. Whole blood was collected first, and PRP and PPP were prepared by centrifugation. PRP was then centrifuged and the platelet concentration was quantitatively adjusted to 10⁹/mL. Water for injection can be added to the quantitative procedure, and the previously quantified PRP can be activated by ultrasonic vibration or added with the activator to achieve platelet purity of 100%, making the growth factor content have a higher effect. The aforementioned activation procedure and the quantitative HCPP were centrifuged and the supernatant was removed. Then, the activated HCPP were mixed with the aforementioned PPP obtained by centrifugation, lyophilized, and sterilized by gamma ray. Finally, a solvent (such as water for injection) of additive 3 was added to form a platelet combination solution 32.

The aforementioned is the preparation process of the platelets-containing composition of the present invention, with the embodiment flow chart of platelet gel and platelet composite solution. The preparation process of the two product forms, where the platelet-poor plasma (PPP) 2 in FIG. 3 refers to the upper layer platelet plasma, and the platelet-rich plasma (PRP) 1 in FIG. 3 refers to the middle layer platelet plasma.

With the aforementioned preparation process, the produced platelets-containing composition 100 has the following characteristics/advantages:
1. The produced platelets-containing composition is lyophilized into a powder state and can be stored at room temperature without special storage conditions. It can be used immediately, eliminating the need for blood collection and preparation every time.
2. The platelets-containing composition can also be prepared without adding additional agent (such as an anticoagulant).
3. The produced platelet gel can also gel at room temperature without being heated before or during use. Since traditional gels require high temperature heating or other additional steps to use (this involves pressing, squeezing, filtering, ultrasonic vibration or freeze and thaw cycle), unlike the present invention, they cannot gel directly at room temperature.
4. The shape of the gel is not limited (for example: a traditional blood collection tube is limited to 10cc in capacity), and a platelets-containing composition with a suitable shape and thickness can be prepared according to the demand.

The present invention's method for preparing the platelets-containing composition has been verified by experiments. The experiments have shown that the platelets-containing composition produced by the present invention's process has more advantageous features.
1. Sterile.
2. No additional complexes, such as gelatin, chitosan, hyaluronic acid.
3. No red blood cells.
4. No separation gel.
5. Containing growth factors.
6. With platelet quantification.
7. No extraneous substances in the preparation process.
8. Can be used as a natural medium for cell culture.
9. 100% platelet content.
10. Gel temperature can be room temperature.
11. No need for additional growth factors.
12. No activator residue.

Application of the present invention:
1. Advantages without additives.
   The preparation process of the platelets-containing composition of the present invention has no additional additives, so the risk of infection can be minimized for patients, and at the same time, no additives can also create low raw materials and added value of preparation costs.
2. Create 100% pure platelets-containing composition with ADP.
   In this present invention, platelet activation is achieved by using adenosine diphosphate (ADP) added to the platelets,, creating and maintaining platelets in an activated state, followed by removal, and then achieving the effect of higher platelet purity and growth factor content.
3. Prepared by collecting the patient's own blood to avoid rejection and toxicity.
   In the present invention, by collecting and preparing the patient's own blood, the use of platelet-containing combination in the present invention avoids issues of rejection and toxicity, making it safe for use within the patient's body platelets-containing composition.
4. A preparation method for large-scale production.
   The preparation process of the present invention allows for the large-scale production of platelets-containing compositions in a single preparation. It can be applied to larger wounds, injury recovery, or mass preparation. The method solves the capacity limitation of 10cc of traditional blood collection tubes (only fixed shape and thickness can be produced), so that the volume of platelet gel formed each time is limited and the shape is difficult to change.
5. Custom shape/thickness can be customized according to the actual use situation.
   The platelets-containing composition of the present invention can be prepared to form shape and thickness according to actual requirement to deal with irregular wounds, or as a repair and protection on a bone. Following the above-mentioned preparation method that is for large-scale production in one preparation, the present invention can prepare platelets-containing compositions that can be formed into shapes and thicknesses that meet actual needs, and can deal with irregular wounds (such as: wounds caused by burns, etc.), bone repair/ protection etc. There is no need to make up small pieces of platelets-containing compositions by yourself, or use unsuitable dressings.
6. Optimizing growth factor sustained release results.

The platelet-containing combination prepared by this present invention platelets-containing composition does not need additional anticoagulants, and the growth factor sustained-release results are better, and can avoid the risk that anticoagulant excess may cause.

The comparison and advantages of the present invention and prior art:
1. The traditional platelet gel can only be applied externally on the patient's wound, so as to achieve the effect of "protection". The platelet gel prepared by the present invention is prepared by collecting the patient's own blood as mentioned above, avoiding the application of rejection and toxicity, then could be that the protective effect can be better, and the patient's wound can be repaired faster.
2. The traditional preparation of platelet composition is to add other ingredients, such as collagen, calcium ions, etc., to the platelet-rich plasma (PRP), and create different forms of liquid, powder, colloid, etc. It cannot rule out allergens, toxicity and other doubts. Platelet count cannot be estimated. The above mentioned 100% pure platelets-containing composition was created with ADP. Through platelet quantification and using ADP to maintain platelets in an activated state, and allows for plasma removal and 100% platelet purity is achieved.
3. The traditional process of forming platelet gel requires additional heating (generally at 37°C), which is inconvenient to use. The platelet gel produced by the present invention does not need to be heated during the use and production stages, so as to avoid the problem that heating destroys the activity of platelets.
4. The traditional platelet gel needs to mix with other substances such as gelatin, chitosan, hyaluronic acid, etc., to form a colloid. The added substances may destroy or impair platelet activity. The preparation process of the present invention does not need to add additional complexes, and can also be completed by making the platelets-containing composition.

Features of the present invention:
1. Feature 1, the platelets-containing composition can be stored at room temperature: the produced platelets-containing composition can be stored at room temperature without being limited by additional storage conditions.
2. Feature 2, no additional complexes added: No need to add additional complexes such as gelatin, chitosan, hyaluronic acid, etc., can also make the platelets-containing composition.
3. Feature 3, no heating required for use: the produced platelet gel does not require to be heated during use and production, and can be gelled and used at room temperature.
4. Feature 4, ability to prepare platelets-containing composition of any shape and thickness: The preparation is not limited by shape (such as the traditional blood collection tube has a capacity limit of 10cc). The platelets-containing composition of suitable shape and thickness can be prepared according to requirements.
5. Feature 5, 100% pure platelets-containing composition: through activation, the platelet concentration reaches 100%.

The platelets-containing composition 100 of the present invention comprises a quantified and activated platelet-rich plasma (PRP) 1 and a platelet-poor plasma (PPP) 2 mixed with the PRP 1. The method for preparing the platelets-containing composition comprises the following steps: collecting whole blood; performing centrifugation to separate platelet-rich plasma (PRP) 1 and platelet-poor plasma (PPP) 2; quantifying and activating the platelet-rich plasma (PRP) 1 into highly concentrated platelet plasma (HCPP); mixing the quantified and activated HCPP and the platelet-poor plasma (PPP) 2; freeze-drying; performing sterilization; completing preparation of the platelets-containing composition. The platelets-containing composition can be stored at room temperature, without adding additional complexes, can be used without heating, and can be prepared in any shape and thickness of high-purity platelets-containing composition. The platelets-containing composition and the preparation process thereof can effectively improve its ease of use, safety, and have the best growth factor sustained-release results. It will greatly expand the application of the industry and have novelty and inventive step.

Although the present invention has been described with reference to the preferred embodiments, it will be apparent to those skilled in the art that a variety of modifications and changes in form and detail may be made without departing from the scope of the present invention defined by the appended claims.

## Claims

1. A platelets-containing composition, comprising:
at least one platelet-rich plasma (PRP) that has been quantified and activated to form a quantified and activated PRP;
at least one platelet-poor plasma (PPP), wherein the at least one PPP is mixed with the quantified and activated platelet-rich plasma (PRP) to form a mixture,
and the mixture is freeze-dried and sterilized to form the platelets-containing composition with high purity and sustained release of growth factors.

2. The platelets-containing composition according to claim 1, wherein the platelets-containing composition is added with an additive according to requirement of use, so that the platelets-containing composition is used in colloid or liquid form.

3. A method for preparing a platelets-containing composition, comprising the following steps:
(1) collecting whole blood;
(2) performing centrifugation to separate platelet-rich plasma (PRP) and platelet-poor plasma (PPP);
(3) quantifying and activating the PRP into highly concentrated platelet plasma (HCPP);
(4) mixing the HCPP and the PPP;
(5) freeze-drying;
(6) performing sterilization;
(7) completing preparation of the platelets-containing composition.

4. The method according to claim 3, wherein in step (7), the platelets-containing composition is added with an additive according to requirement of use, so that the platelets-containing composition is used in colloid or liquid form.

5. The method according to claim 4, wherein the additive is in the range of 0.01-10% Ca²⁺, and a platelet gel is formed.

6. The method according to claim 4, wherein the additive added per milliliter (mL) of the platelets-containing composition is in the range of 0.05-9.75% Ca²⁺.

7. The method according to claim 4, wherein the additive added per milliliter (mL) of the platelets-containing composition is in the range of 0.1-9% Ca²⁺.

8. The method according to claim 4, wherein the additive added per milliliter (mL) of the platelets-containing composition is the group consisting of: 0.01%, 0.02%, 0.03%, 0.04%, 0.05%, 0.06%, 0.07%, 0.08%, 0.09%, 0.1%, 0.11%, 0.12%, 0.13%, 0.14%, 0.15%, 0.16%, 0.17%, 0.18%, 0.19%, 0.2%, 0.21%, 0.22%, 0.23%, 0.24%, 0.25%, 0.26%, 0.27%, 0.28%, 0.29%, 0.3%, 0.31%, 0.32% , 0.33%, 0.34%, 0.35%, 0.36%, 0.37%, 0.38%, 0.39, 0.4%, 0.41%, 0.42%, 0.43%, 0.44%, 0.45%, 0.46%, 0.47%, 0.49%, 0.5% , 0.51%, 0.52%, 0.53%, 0.54%, 0.55%, 0.56%, 0.57%, 0.58%, 0.59%, 0.6%, 0.61%, 0.62%, 0.63%, 0.64%, 0.65%, 0.66%, 0.67 %, 0.68%, 0.69%, 0.7%, 0.71%, 0.72%, 0.73%, 0.74%, 0.75%, 0.76%, 0.77%, 0.78%, 0.79%, 0.8%, 0.81%, 0.82%, 0.83%, 0.84%, 0.85%, 0.86%, 0.87%, 0.89%, 0.9%, 1%, 1.25%, 1.5%, 1.75%, 2%, 2.25%, 2.5%, 2.75%, 3%, 3.25%, 3.5% , 3.75%, 4%, 4.25%, 4.5%, 4.75%, 5%, 5.25%, 5.75%, 6%, 6.25%, 6.5%, 6.75%, 7%, 7.25%, 7.5%, 8.75%, 9 %, 9.25%, 9.5%, 9.75%, and 10%.

9. The method according to claim 4, wherein the gel is prepared in shape and thickness of actual requirement, making it suitable for irregular wounds, or as a repair and protection on a bone.

10. The method according to claim 4, wherein the additive is a solvent, and a platelet composite solution is formed.

11. The method according to claim 3, wherein quantifying the PRP in step 3 is through centrifugation and adding water for injection to adjust platelet concentration to 10⁹/mL.

12. The method according to claim 11, wherein the quantification of the platelet-rich plasma (PRP) is achieved by adding sterile normal saline, 0.45% NaCl, 4.5% hypertonic saline, sterile hot spring water or isotonic saline as a substitute for water for injection.

13. The method according to claim 3, wherein the activating platelet-rich plasma (PRP) is by adding an activator or by ultrasonic vibration.

14. The method according to claim 3, wherein the activating the platelet-rich plasma (PRP) is by adding an activator, and supernatant is removed by centrifugation to mix with the platelet-poor plasma (PPP).

15. The method according to claim 3, wherein the activating the platelet-rich plasma (PRP) is through ultrasonic vibration to mix the HCPP and the platelet-poor plasma (PPP).
